(19) Europäisches Patentamt
European Patent Office
Office européen des brevets

(11) Publication number: 0 192 317 B1

(12) **EUROPEAN PATENT SPECIFICATION**

(45) Date of publication of patent specification: 28.02.90

(51) Int. Cl.[5]: C 07 D 233/64, A 61 K 31/415

(21) Application number: 86300076.6

(22) Date of filing: 07.01.86

(54) **Homocarnosine or acylhomocarnosine salts.**

(30) Priority: 19.01.85 JP 8243/85

(43) Date of publication of application: 27.08.86 Bulletin 86/35

(45) Publication of the grant of the patent: 28.02.90 Bulletin 90/09

(84) Designated Contracting States: BE CH DE FR GB IT LI NL SE

(56) References cited:
FR-A-2 464 943

CHEMICAL ABSTRACTS, vol. 77, no. 1, 3rd July 1972, page 69, no. 672k, Columbus, Ohio, US; M.J. TURNBULL et al.: "Pharmacological properties of homocarnosine", & ARCH. INT. PHARMACODYN. THER. 1972, 196(1), 127-32

(73) Proprietor: HAMARI YAKUHIN KOGYO KABUSHIKI KAISHA also known as HAMARI CHEMICALS, LTD.
4-29, Kunijima 1-chome Higashi-yodogawa-ku
Osaka 533 (JP)

(72) Inventor: Takaya, Masahiro
121-11, Yukihata-sannotsubo Oaza
Shiga 520-23 (JP)

(74) Representative: Burford, Anthony Frederick et al
W.H. Beck, Greener & Co. 7 Stone Buildings Lincoln's Inn
London WC2A 3SZ (GB)



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a new homocarnosine or acylhomocarnosine zinc or aluminum salt. More particularly, this invention relates to a homocarnosine or acylhomocarnosine aluminum or zinc salt of the general formula:

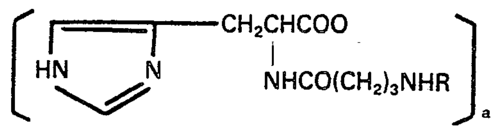

$M(OH)_b$ (I)

wherein R represents a hydrogen atom or an acetyl or benzoyl group; M represents a zinc atom or an aluminum atom; *a* denotes 1, 2 or 3; and *b* denotes 0, 1 or 2, provided that when *a* denotes 1, *b* denotes 0 and M represents zinc, a hydrogen atom is omitted from the homocarnosine or acylhomocarnosine residue. These new compounds [I] have excellent antiulcer and wound healing-promoting actions.

The conventional drug therapy for peptic ulcer involves administration of antacids having gastric mucosa-protecting and tissue-repairing actions, such as bismuth drugs, aluminum preparations, L-glutamine preparations, etc. However, these drugs generally have the disadvantage that they are not sufficiently effective, although the side effects they induce are relatively mild.

Recently, with an increasing incidence of peptic ulcer, various studies on therapeutic drugs for the disease have been undertaken and particularly as the mechanism of onset of peptic ulcer has been made increasingly clear, therapeutic drugs antagonizing the ulcer such as histamine $H_2$-receptor antagonists, gastrin antagonists and autonomic nerve blocking agents have been studied in detail. Actually, some drugs developed along these lines have already been on the market.

However, while these antagonists and blockers are generally potent, they produce severe and diverse side effects, thus presenting various problems from the standpoint of drug safety.

Further, in the field of dermatological drugs for external use, there is the axiom that "spontaneous healing cannot be accelerated" and, for this reason, little study has been undertaken on the action of drugs to promote spontaneous healing of wounds.

In view of the above drawbacks and problems, the present inventor conducted an intensive study to develop a drug which would protect the gastric mucosa and repair the damaged tissue, cause little side effect, produce remarkable therapeutic effects and be useful as a therapeutic agent for peptic ulcer or/and an agent for promoting the spontaneous healing of wounds.

It has been reported in FR—A—2464943 that acylcarbosine aluminium salts can be used in the treatment of gastric ulcers.

As a candidate drug, the present inventor paid attention to homocarbosine, a compound which had not been much studied so far, and its acyl derivatives but these these compounds as such were found to be inadequate in therapeutic action on peptic ulcer or in the action to promote spontaneous healing of wounds and do not warrant development as drugs.

In an attempt to overcome the above-mentioned problems, the present inventor synthesized a large number of derivatives of homocarnosine and investigated their therapeutic activity and other properties. As a result, it was found that the zinc or aluminum salts of homocarnosine and acylhomocarosines which may be represented by the above general formula [I] produce excellent therapeutic effects against peptic ulcer and also possess high activty to promote spontaneous healing of wounds and that they cause only mild side effects. Thus, it was found that the above-mentioned problems can be overcome by converting homocarnosine or an acylhomocarnosine into the corresponding zinc or aluminum salt. This invention has been predicated on the above finding.

This invention is, therefore, directed to a homocarnosine salt of the general formula:

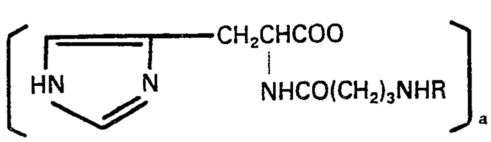

$M(OH)_b$ (I)

wherein R represents hydrogen, acetyl or benzoyl; M represents a zinc atom or an aluminum atom; *a* denotes 1, 2 or 3; and *b* denotes 0, 1 or 2, provided that when *a* denotes 1, *b* denotes 0 and M represents zinc, a hydrogen atom is omitted from the homocarnosine or acylhomocarnosine residue. The homocarnosine or acylhomocarnosine zinc salt according to this invention can be produced easily and in quantitative yield by reacting homocarnosine or an acylhomocarnosine with a zinc compound such as a zinc halide, zinc sulfate or zinc hydroxide in the presence or absence of a basic agent such as sodium hydroxide, potassium hydroxide or a sodium alkoxide in a hydrophilic or hydrophobic solvent, water, or a mixture of water and such a solvent. In a homocarnosine or acylhomocarnosine zinc salt, it is considered that the homocarnosine or acylhomocarnosine molecule is usually combined with zinc atom in the state of

lacking 2 hydrogen atoms of the molecule. The homocarnosine or acylhomocarnosine aluminum salt can also be produced in quantitative yield by reacting homocarnosine or an acylhomocarnosine with an aluminum alkoxide in a hydrophilic or hydrophobic solvent, water, or a mixture of water and such a solvent. In either case, the reaction is generally conducted at room temperature or under mild heating for fractions of hour to several hours. The reaction product thus obtained can be worked up and purified in the per se conventional manner, for example by extraction, recrystallization, concentration, filtration, aqueous washing, activated charcoal treatment, column chromatography, resin treatment, etc.

An acylhomocarnosine can be easily produced by acylating homocarnosine with, for example, acetic anhydride, an acetyl halide, a benzoyl halide or the like in the per se conventional manner. The homocarnosine or acylhomocarnosine for this purpose may be in any of its D-form, L-form and DL-form.

The compounds according to this invention have excellent antiulcer and wound healing-promoting actions and are, therefore, of value as antiulcer drugs or dermatological therapeutic drugs for external application, therapeutic drugs for stromatitis, therapeutic drugs for hemorrhoids, etc.

These compounds are used orally in such dosage forms as tablets, capsules, granules, fine granules, syrups, etc. or non-orally as injections, ointments, powders, liquids, etc. For oral administration, the compounds are used generally in a daily dose of 20 mg to 2 g. According to the disease, patient's condition and therapeutic procedure, they can also be used in combination with other drugs.

1. Antiulcer activity

(a) Water-immersion stress ulcer

Male JCL—SD strain rats aged 7.5 weeks and weighing 220—250 g were used in groups of 7 animals. After a fasting period of 24 hours, 400 mg/kg of each test compound was orally administered to each animal, and after 30 minutes, the animal was immobilized in a stress cage and was immersed in 23°C water down to the level of the thorax for 7 hours. After this stress loading, the rats were exsanguinated by decapitation and the stomach was excised and fixed in 1% formalin for 15 minutes. Then, an incision was made from the pyrolus along the greater curvature and the lengths of the lesions formed in the body of the stomach were measured using a dissecting microscope (with a scale graduated in 1/100 cm). The sum of the lengths was used as the ulcer index.

The results of the experiment are shown in Table I.

TABLE I
Inhibition of water-immersion stress ulcer

| Test compound | Ulcer Index (mm) | Inhibition (%) |
|---|---|---|
| No drug | 10.24 | 0 |
| Homocarnosine | 8.81 | 13.9 |
| N-Acetyl-L-Homocarnosine | 9.95 | 6.7 |
| Homocarnosine Zn | 3.85 | 62.4 |
| (Homocarnosine)$_2$ Zn | 6.34 | 38.0 |
| N-Acetyl-L-Homocarnosine Zn | 7.06 | 31.0 |
| (N-Acetyl-L-Homocarnosine)$_2$ Zn | 8.32 | 18.7 |
| Homocarnosine Al(OH)$_2$ | 7.10 | 30.6 |
| (Homocarnosine)$_2$ AlOH | 7.76 | 24.2 |
| (Homocarnosine)$_3$ Al | 8.24 | 19.5 |
| N-Acetyl-L-Homocarnosine Al(OH)$_2$ | 5.40 | 47.2 |
| (N-Acetyl-L-Homocarnosine)$_3$ Al | 8.46 | 17.3 |
| Carnosine Zn (reference drug) | 4.61 | 54.9 |
| Cimetidine (reference drug) | 4.02 | 60.7 |
| Basic aluminum sucrose sulfate (reference drug) | 8.54 | 16.6 |

(b) Pyrolic ligation-induced ulcer

Male Wistar strains rats weighing about 300 g were deprived of food for 20 hours, with provision against fecal feeding, and used in groups of 8 animals. The pyrolus of each animal was ligated under ether anesthesia according to the method described by Shay et al. [H. G. Shay, S. A. Komarow, S. S. Felds, D. Meranze, M. Gruenstein, and H. Siplet, Gastroenterology, 5, 43 (1945)], and 300 mg/kg of each test compound was immediately administered intraduodenally. The incision was sutured and 13 hours after drug administration the rat was sacrificed by cervical dislocation. The stomach was excised, slighly inflated by an injection of 1% formalin solution, immersed in the same solution for 15 minutes to fix the gastric wall, and then opened along the greater curvature. The ulcer in the fore-stomach was measured under a dissecting microscope to calculate the ulcer index and the efficacy of the drug was expressed in terms of percent ulcer index. The results are shown in Table II.

TABLE II

Percent inhibition of pyrolic ligation-induced ulcer

| Test compound | Inhibition (%) |
|---|---|
| No drug | 0 |
| Homocarnosine | 6.2 |
| N-Acetyl-L-Homocarnosine | 2.4 |
| Homocarnosine Zn | 46.2 |
| N-Acetyl-L-Homocarnosine Zn | 22.1 |
| Homocarnosine $Al(OH)_2$ | 11.2 |
| N-Acetyl-L-Homocarnosine $Al(OH)_2$ | 38.7 |
| Carnosine Zn (reference drug) | 39.2 |
| Basic aluminum sucrose sulfate (reference drug) | 4.7 |

As apparent from the foregoing, homocarnosine zinc salt was the most potent among the compounds tested.

2. Wound healing-promoting activity

(Method)

Male rats weighig about 180 g were used in groups of 9 animals. The hair on the back of each rat was clipped with an electric clipper and under ether anesthesia, a wound (20 mm long) was made with a chisel having a 20 mm edge in two positions of the cropped back. A suspension of each test compound (10 g) in 20 ml of peanut oil was applied to the wound in a volume of 0.05 ml per dose once daily for 10 consecutive days. After 10 days, the animal was bled to death by decapitation and the skin of the back was peeled until the wound was separated. The tension (grams) required for separating the wound was measured as an indicator of drug efficacy.

(Results)

The results are shown in Table III. It is seen that homocarnosine Zn is particularly excellent in wound healing-promoting effect.

TABLE III

Wound healing — promoting effect

| Test compound | Tension (g) |
|---|---|
| Peanut oil (control) | 198.44 |
| Homocarnosine | 218.78 |
| N-Acetyl-L-homocarnosine | 207.45 |
| (Homocarnosine) Zn | 249.36 |
| (N-Acetyl-L-homocarnosine) Zn | 233.64 |
| (Homocarnosine) $Al(OH)_2$ | 220.28 |
| (N-Acetyl-L-homocarnosine) $Al(OH)_2$ | 203.57 |

Acute toxicity study

Wistar strain rats of either sex weighing 150 to 200 g were used in groups of 7 animals. The test compounds (L-homocarnosine, N-acetyl-L-homocarnosine and the compounds of Examples 1 to 9) were respectively administered orally in a dose of 5 g/kg each and the animals were observed for 7 days. No death occurred in any of the groups, indicating that all of these compounds are very low in toxicity.

The foregoing results may be summarized as follows. The novel compounds (I) according to this invention have excellent antiulcer and wound healing-promoting actions and are very low in toxicity. Therefore, these compounds are of great value as drugs for the treatment of ulcer which usually requires a long time to cure or as other drugs such as dermatological therapeutic drugs for external use. Further, by taking advantage of their pharmacological actions, these compounds may be used as drugs for the treatment of stomatitis, drugs for the treatment of hemorrhoids, and so on.

Example 1

To a solution of L-homocarnosine (4.80 g) in methanol (27 ml) containing 28% methanolic sodium methoxide (7.71 g) was added a solution prepared from 96% zinc chloride (2.84 g) and water (1.5 ml) and methanol (50 ml) with stirring. The reaction mixture was stirred at room temperature for 2 hours, and then the resulting precipitate was filtered off, washed fully with water and dried at 100°C for 5 hours to give L-homocarnosine zinc salt (6 g) as colorless powder, m.p. 300°C over.

IR (Nujol*) $cm^{-1}$: 3275, 1620, 1240, 1120, 1045, 980.
$[\alpha]_D^{20}$: +13.27 (c = 0.8, 0.36% HCl)
Zinc content (EDTA Chelate method): 21.73%
Water content (Karl-Fischer method): 0.71%
Elemental analysis (%):

| | | | |
|---|---|---|---|
| Calcd. for $C_{10}H_{14}N_4O_3Zn$: | C, 39.52; | H, 4.61; | N, 18.44 |
| Found: | C, 39.36; | H, 4.48; | N, 18.32 |

Example 2

A mixture of L-homocarnosine (12 g) and zinc hydroxide (2.48 g) in water (150 ml) was stirred at 60°C for 2 hours. From the reaction mixture, the solvent was evaporated to dryness under reduced pressure and the residue was further dried at 80°C for 10 hours to give $(L\text{-homocarnosine})_2$ zinc salt (13.2 g) as colorless powder.

m.p. gradually decomposes at 255°C over.
IR (Nujol) $cm^{-1}$: 3250, 1620, 1570, 1265, 1225, 1160, 1115, 1000, 975.
Nitrogen content (Kjeldahl method): 19.07%
Zinc content (EDTA Chelate method): 11.13%
Water content (Karl-Fischer method): 2.14%

Example 3

To a solution of N-acetyl-L-homocarnosine (2.82 g) in methanol (40 ml) containing 28% methanolic sodium methoxide (3.86 g) was added a solution prepared from 96% zinc chloride (1.42 g), water (1.42 g) and methanol (6 ml) with stirring. The mixture was stirred at room temperature for 2 hours. The resulting

* Nujol is a Trade Mark

precipitate was collected by filtration and dried at 70°C for 8 hours to give N-acetyl-L-homocarnosine zinc salt (4 g) as colorless powder.

m.p. 250—263°C (decomp.)

IR (Nujol) $cm^{-1}$: 3275, 1630, 1540, 1300, 1240, 1195, 1115, 1045, 975.

Nitrogen content (Kjeldahl method): 16.04%

Zinc content (EDTA Chelate method): 19.47%

Water content (Karl-Fischer method): 6.51%

Example 4

To a stirred solution of zinc hydroxide (1.19 g) in water (50 ml) was added a solution previously prepared from N-acetyl-L-homocarnosine (5.64 g) and water (40 ml) at 50—55°C. The mixture was further stirred at 50—55°C for 5 hours. After cooling, the reaction mixture was filtered to remove insoluble substance and the filtrate was concentrated under reduced pressure. To the residue, isopropyl alcohol (70 ml) was added and the resulting precipitate was collected by filtration and dried at 95°C for 7 hours to give $(N\text{-acetyl-L-homocarnosine})_2$ zinc salt (6.5 g) as colorless powder.

m.p. 187—195°C (decomp.)

IR (Nujol) $cm^{-1}$: 3275, 1630, 1540, 1300, 1200, 1115, 970.

Nitrogen content (Kjeldahl method): 17.91%

Zinc content (EDTA Chelate method): 10.54%

Water content (Karl-Fischer method): 0.96%

Example 5

To a solution of isopropyl alcohol (200 ml) containing aluminum isopropoxide (20.42 g) was added L-homocarnosine (24.02 g) with stirring at 60°C. After the mixture was stirred at 60°C for 30 minutes, water (3.6 ml) was added and then the mixture was further stirred at that temperature for 2 hours. After cooling, the resulting precipitate was collected on a filter by suction and dried at 95°C for 10 hours to give L-homocarnosine aluminum salt (30.5 g) as colorless powder.

m.p. gradually decomposes at 220°C over.

IR (Nujol) $cm^{-1}$: 3300, 1630, 1600, 1545, 1130, 1290, 1275, 1225, 1185, 1155, 1120, 1090, 985.

Aluminum content ($Al_2O_3$ weight method): 8.84%

Water content (Karl-Fischer method): 2.82%

Elemental analysis (%):

| | | | |
|---|---|---|---|
| Calcd. for $C_{10}H_{15}N_4O_3Al(OH)_2$: | C, 39.70; | H, 5.62; | N, 18.53 |
| Found: | C, 39.57; | H, 5.77; | N, 18.36 |

Example 6

To a stirred solution of isopropyl alcohol (100 ml) containing aluminum isopropoxide (10.2 g) was added L-homocarnosine (24.02 g) at 50°C with stirring. After the mixture was stirred 50°C for 10 minutes, water (1.8 ml) was added and the mixture was refluxed with stirring for 1 hour. After cooling, the resulting precipitate was collected by filtration and dried at 95°C for 10 hours to give L-homocarnosine aluminum salt (27.1 g) as colorless powder.

m.p. gradually decomposes at 220°C over.

IR (Nujol) $cm^{-1}$: 3300, 1630, 1600, 1545, 1127, 1290, 1275, 1225, 1185, 1155, 1093, 985.

Aluminum content ($Al_2O_3$ weight method): 5.03%

Water content (Karl-Fischer method): 1.78%

Elemental analysis (%):

| | | | |
|---|---|---|---|
| Calcd. for $C_{10}H_{15}N_4O_3AlOH$: | C, 45.85; | H, 5.92; | N, 21.39 |
| Found: | C, 46.39; | H, 5.97; | N, 20.85 |

Example 7

To a stirred solution of dry isopropyl alcohol (60 ml) containing aluminum isopropoxide (6.13 g) was added L-homocarnosine (21.62 g) at 50°C. The mixture was stirred at 60°C for 2 hours and the solvent in the mixture was evaporated in vacuo. The resulting residue was dried at 80°C for 8 hours to give L-homocarnosine aluminum salt (22.0 g) as colorless powder.

m.p. gradually decomposes at 215°C over.

IR (Nujol) $cm^{-1}$: 3300, 1630, 1597, 1545, 1125, 1290, 1275, 1225, 1185, 1155, 1090, 987.

Aluminum content ($Al_2O_3$ weight method): 3.47%

Water content (Karl-Fischer method): 1.97%

Elemental analysis (%):

| | | | |
|---|---|---|---|
| Calcd. for $(C_{10}H_{15}N_4O_3)_3Al$: | C, 48.34; | H, 6.04; | N, 22.56 |
| Found: | C, 47.83; | H, 5.96; | N, 22.18 |

Example 8

To a stirred solution of isopropyl alcohol (200 ml) containing aluminum isopropoxide (20.4 g) was added N-acetyl-L-homocarnosine (28.2 g) at room temperature. After the mixture was stirred at room temperature for 20 minutes, water (3.6 g) was added and then heated under reflux for 1 hour with stirring.

The solvent in the reaction mixture was removed by evaporation and the resulting residue was dried under reduced pressure in a desiccator at room temperature for several days to give N-acetyl-L-homocarnosine aluminum salt as colorless powder.

m.p. 231—236°C (decomp.)
IR (Nujol) $cm^{-1}$: 3255, 1635, 1300, 1185, 1105, 970.
Aluminum content ($Al_2O_3$ weight method): 7.96%
Water content (Karl-Fischer method): 3.84%
Elemental analysis (%):

| | | | |
|---|---|---|---|
| Calcd. for $C_{12}H_{17}N_4O_4Al(OH)_2$: | C, 41.83; | H, 5.51; | N, 16.26 |
| Found: | C, 40.98; | H, 5.63; | N, 15.87 |

Example 9

A mixture of N-acetyl-L-homocarnosine (42.3 g) and aluminum isopropoxide (10.2 g) in dry isopropyl alcohol (200 ml) was refluxed for 1 hour with stirring. After evaporating the solvent in the reaction mixture to dryness, the reamining residue was dried under reduced pressure in a desiccator at room temperature for several days to give N-acetyl-L-homocarnosine aluminum salt as colorless powder.

m.p. 155—165°C (decomp.)
IR (Nujol) $cm^{-1}$: 3255, 1635, 1550, 1300, 1185, 1105, 1035, 970.
Aluminum content ($Al_2O_3$ weight method): 3.18%
Water content (Karl-Fischer method): 1.79%
Elemental analysis (%):

| | | | |
|---|---|---|---|
| Calcd. for $(C_{12}H_{17}N_4O_4)Al$: | C, 49.61; | H, 5.85; | N, 19.29 |
| Found: | C, 49.02; | H, 5.72; | N, 18.74 |

**Claims**

1. A homocarnosine or acylhomocarnosine aluminum or zinc salt of the formula:

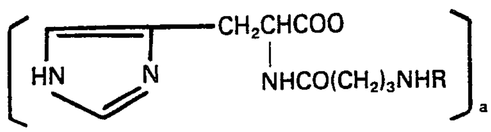 $M(OH)_b$ (I)

wherein R represents hydrogen, acetyl or benzoyl; M represents a zinc atom or an aluminum atom; *a* denotes 1, 2 or 3; and *b* denotes 0, 1 or 2, provided that when *a* denotes 1, *b* denotes 0 and M represents zinc, a hydrogen atom is omitted from the homocarnosine or acylhomocarnosine.

2. A homocarnosine or acylhomocarnosine zinc salt as claimed in Claim 1, wherein said salt has the formula:

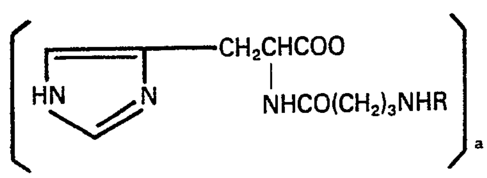 Zn

wherein R represents hydrogen, acetyl or benzoyl; and *a* denotes 1 or 2, provided that, when *a* denotes 1, an unspecifiable hydrogen is omitted from the homocarnosine or acylhomocarnosine residue.

3. A homocarnosine or acylhomocarnosine aluminum salt as claimed in Claim 1, wherein said salt has the formula:

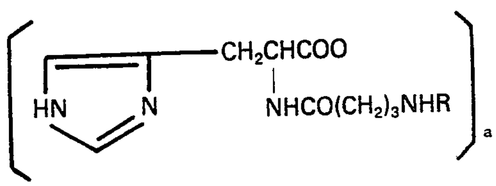 $Al(OH)_b$

wherein R represents hydrogen, acetyl or benzoyl; *a* denotes 1, 2 or 3; and *b* denotes 0, 1 or 2.

4. An acylhomocarnosine aluminum or zinc salt as claimed in any one of the preceding claims, wherein R represents acetyl.

5. A homocarnosine or acylhomocarnosine zinc salt selected from L-homocarnosine Zn, $(L\text{-homocarnosine})_2$ Zn, N-acetyl-L-homocarnosine Zn and $(N\text{-acetyl-L-homocarnosine})_2$ Zn.

6. A homocarnosine or acylhomocarnosine aluminum salt selected from L-homocarnosine $Al(OH)_2$, $(L\text{-homocarnosine})_2$ AlOH, N-acetyl-L-homocarnosine $Al(OH)_2$ and $(N\text{-acetyl-L-homocarnosine})_2$ AlOH.

7. Homocarnosine Zn.

8. A pharmaceutical composition comprising a homocarnosine or acylhomocarnosine aluminum or zinc salt as claimed in any one of the preceding claims in admixture, or otherwise associated with, a pharmaceutically acceptable diluent or carrier.

9. A homocarnosine or acylhomocarnosine aluminum or zinc salt as claimed in any one of Claims 1 to 7 for use in the treatment of ulcers.

10. A homocarnosine or acylhomocarnosine aluminum or zinc salt as claimed in any one of Claims 1 to 7 for use in promoting wound healing.

11. The use of a homocarnosine or acylhomocarnosine aluminum or zinc salt as claimed in any one of Claims 1 to 7, in the manufacture of a medicament for the treatment of ulcers.

12. The use of a homocarnosine or acylhomocarnosine aluminum or zinc salt as claimed in any one of Claims 1 to 7, in the manufacture of a medicament for promoting wound healing.

**Patentansprüche**

1. Homocarnosin- oder Acylhomocarnosin-Aluminium- oder Zinksalz der folgenden allgemeinen Formel

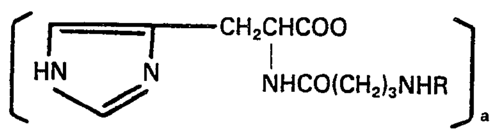 $M(OH)_b$ (I)

worin

R für ein Wasserstoffatom, einen Acetyl- oder Benzoyl-Rest steht,

M für ein Zinkatom oder ein Aluminiumatom steht;

a für 1, 2 oder 3 steht; und

b für 0, 1 oder 2 steht,

mit der Maßgabe, daß, wenn a für 1, b für 0 und M für Zink steht, ein Wasserstoffatom des Homocarnosins oder Acyl homocarnosins fehlt.

2. Homocarnosin- oder Acylhomocarnosin-Zinksalz gemäß Anspruch 1, wobei das Salz folgende allgemeine Formel besitzt:

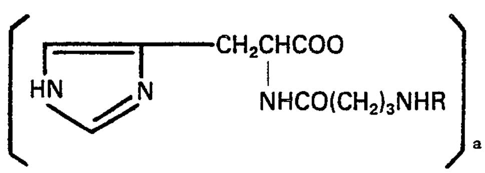 Zn

worin

R für ein Wasserstoffatom, einen Acetyl- oder Benzoyl-Rest steht; und

a für 1 oder 2 steht,

mit der Maßgabe, daß, wenn a für 1 steht, ein unbestimmtes Wasserstoffatom des Homocarnosin- oder Acylhomocarnosin-Restes fehlt.

3. Homocarnosin- oder Acylhomocarnosin-Aluminiumsalz gemäß Anspruch 1, wobei das Salz folgende allgemeine Formel besitzt:

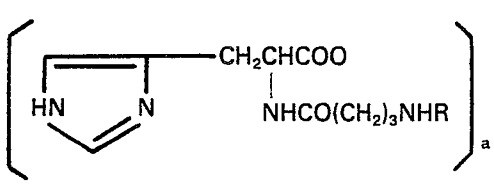 $Al(OH)_b$

worin

R für ein Wasserstoffatom, einen Acetyl- oder Benzoyl-Rest steht;

a für 1, 2 oder 3 steht; und

b für 0, 1 oder 2 steht.

4. Acylhomocarnosin-Aluminium- oder Zinksalz nach einem der vorhergehenden Ansprüche, worin R für Acetyl steht.

5. Homocarnosin- oder Acylhomocarnosin-Zinksalz, ausgewählt unter L-Homocarnosin-Zn, $(L\text{-}Homocarnosin)_2$-Zn, N-Acetyl-L-homocarnosin-Zn und $(N\text{-}Acetyl\text{-}L\text{-}homocarnosin)_2$-Zn.

6. Homocarnosin- oder Acylhomocarnosin-Aluminiumsalz, ausgewählt unter L-Homocarnosi-$Al(OH)_2$, $(L\text{-}Homocarnosin)_2$-AlOH, N-Acetyl-L-homocarnosin-$Al(OH)_2$ und $(N\text{-}Acetyl\text{-}L\text{-}homocarnosin)_2$-AlOH.

7. Homocarnosin-Zn.

8. Pharmazeutisches Gemisch, welches ein Homocarnosin- oder Acylhomocarnosin-Aluminium- oder

Zinksalz nach einem der vorhergehenden Anspruche im Gemisch, oder in anderer Weise assoziiert, mit einem pharmazeutisch verträglichen Verdünnungsmittel oder Träger enthält.

9. Homocarnosin- oder Acylhomocarnosin-Aluminium- oder Zinksalz nach einem der Ansprüche 1 bis 7 zur Verwendung bei der Ulkus-Behandlung.

10. Homocarnosin- oder Acylhomocarnosin-Aluminium- oder Zinksalz nach einem der Ansprüche 1 bis 7 zur Verwendung bei der Förderung der Wundheilung.

11. Verwendung eines Homocarnosin- oder Acylhomocarnosin-Aluminium- oder Zinksalzes nach einem der Ansprüche 1 bis 7 zur Herstellung von Medikamenten zur Ulkus-Behandlung.

12. Verwendung eines Homocarnosin- oder Acylhomocarnosin-Aluminium- oder Zinksalzes nach einem der Ansprüche 1 bis 7 zur Herstellung eines Medikamentes zur Förderung der Wundheilung.

**Revendications**

1. Sel d'aluminium ou de zinc d'homocarnosine ou d'acylhomocarnosine de formule

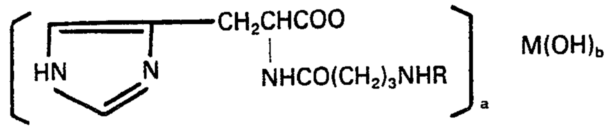 (I)

dans laquelle R représente un atome d'hydrogène ou un groupement acétyle ou benzoyle; M représente un atome de zinc ou d'aluminium; a désigne 1, 2 ou 3; b désigne 0, 1 ou 2, pourvu que, quand a désigne 1, b désigne 0 et M représente le zinc, un atome d'hydrogène soit omis de l'homocarnosine ou de l'acylhomocarnosine.

2. Sel de zinc d'homocarnosine ou d'acylhomocarnosine, ledit sel ayant la formule

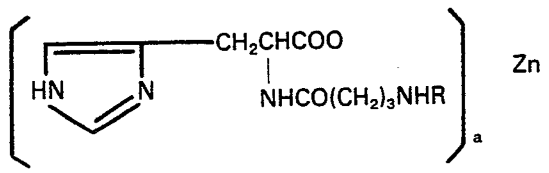

dans laquelle R représente un atome d'hydrogène ou un groupement acétyle ou benzoyle; et a désigne 1 ou 2, pourvu que, quand a désigne 1, un hydrogène non identifiable soit omis du reste homocarnosine ou acylhomocarnosine.

3. Sel d'aluminium d'homocarnosine ou d'acylhomocarnosine selon la revendication 1, ledit sel ayant la formule

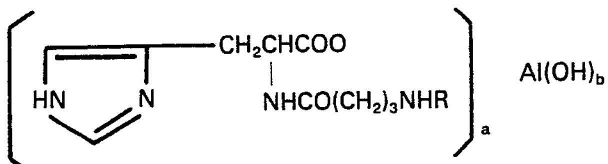

dans laquelle R représente un atome d'hydrogène ou un groupement acétyle ou benzoyle; a désigne 1, 2 ou 3 et b désigne 0, 1 ou 2.

4. Sel d'aluminium ou de zinc d'acylhomocarnosine selon l'une quelconque des revendications 1 à 3 dans lequel R représente un groupement acétyle.

5. Sel de zinc d'homocarnosine ou d'acylhomocarnosine choisi entre L-homocarnosine-Zn, $(L\text{-homocarnosine})_2$-Zn, N-acétyl-L-homocarnosine-Zn et $(N\text{-acétyl-L-homocarnosine})_2$-Zn.

6. Sel d'aluminium d'homocarnosine ou d'acylhomocarnosine choisi entre L-homocarnosine-$Al(OH)_2$, $(L\text{-homocarnosine})_2$-AlOH, N-acétyl-L-homocarnosine-$Al(OH)_2$ et $(N\text{-acétyl-L-homocarnosine})_2$-AlOH.

7. Homocarnosine-Zn.

8. Composition pharmaceutique comprenant un sel d'aluminium ou de zinc d'homocarnosine ou d'acylhomocarnosine selon l'une quelconque des revendications précédentes, en mélange ou associé de toute autre façon avec un diluant ou support acceptable sur le plan pharmaceutique.

9. Sel d'aluminium ou de zinc d'homocarnosine ou d'acylhomocarnosine selon l'une quelconque des revendications 1 à 7, destiné à être utilisé dans le traitement des ulcères.

10. Sel d'aluminium ou de zinc d'homocarnosine ou d'acylhomocarnosine selon l'une quelconque des revendications 1 à 7, destiné à être utilisé pour promouvoir la guérison des blessures.

11. Utilisation d'un sel d'aluminium ou de zinc d'homocarnosine ou d'acylhomocarnosine selon l'une quelconque des revendications 1 à 7, dans la fabrication d'un médicament pour le traitement des ulcères.

12. Utilisation d'un sel d'aluminium ou de zinc d'homocarnosine ou d'acylhomocarnosine selon l'une quelconque des revendications 1 à 7, dans la fabrication d'un médicament promouvant la guérison des blessures.